# EUROPEAN PATENT APPLICATION

(11) **EP 1 813 311 A1**
(43) Date of publication of application: **01.08.2007**
(21) Application number: 05025718.7
(22) Date of filing: 25.11.2005
(51) Int. Cl.: A61Q 19/00, A61K 8/06, A61K 8/31, A61K 8/37, A61K 8/44, A61K 8/60, A61K 8/92

(54) **Oil-in-water emulsions based on special emulsifiers**

(71) Applicant: Cognis IP Management GmbH, 49589 Düsseldorf (DE)
(72) Inventor: Soms, Jörg, 40476 Düsseldorf (DE); Kawa, Rolf, 40789 Monheim (DE); Eichhorn, Stephan, 64579 Gernsheim (DE); Urban, Andrea, 67069 Ludwigshafen (DE)
(74) Representative: Reinhardt, Jürgen

(57) **Abstract**

The present invention relates to an 0/W emulsion containing
(a) at least one polyol poly-12-hydroxystearate
(b) at least one further non-ionic emulsifier
(c) at least one anionic co-emulsifier
(d) oil, where at least 75 weight-% of the oil has a polarity of ≥ 20 mN/m
(e) 10 to 30 weight -% water based on the total weight of the emulsion
(f) optionally at least one consistency regulator
(g) optionally at least one humectant
- whereas the total amount of emulsifiers [=(a) plus (b) plus (c)] is between 4 and 20 weight-%, preferably between 6 and 16 weight-%, based on the total weight of the emulsion.

The present invention also relates to the use of the emulsion for the preparation of a cosmetic composition.

## Description

### FIELD OF THE INVENTION

This invention relates to special, very mild emulsions which may be used as body care preparations and, in particular, for impregnating and wetting utility and hygienic tissue substrates, e.g. paper.

### BACKGROUND ART

The generic term "paper" encompasses about 3000 different types and articles which can differ, sometimes considerably, in their applications and their properties. The production of paper involves the use of numerous additives among the most important of which are fillers (for example chalk or kaolin) and binders (for example starch). For tissues and hygienic papers, which come into relatively close contact with the human skin, there is a particular need for an agreeable soft feel which is normally given to the paper by careful selection of the fibers and, in particular, by a high percentage of fresh mechanical wood pulp or cellulose. However, in the interests of economic paper manufacture and from the ecological perspective, it is desirable to use large amounts of inferior-quality wastepaper. Unfortunately, this means that the softness of the paper is significantly reduced which is troublesome to users and can even lead to irritation of the skin, particularly with frequent use.

Accordingly, there has been no shortage of attempts in the past to treat tissue papers by impregnation, coating or other surface treatments in such a way that a more agreeable soft feel is achieved. Special lotions and emulsions that are easy to apply to the paper and do not adversely affect its structure have to be developed for this purpose.

International patent application WO 95/35411 relates to tissue papers coated with softening compositions which contain 20 to 80% by weight of a water-free emollient (mineral oils, fatty acid esters, fatty alcohol ethoxylates, fatty acid ethoxylates, fatty alcohols and mixtures thereof), 5 to 95% by weight of an "immobilizing" agent for the emollient (fatty alcohols, fatty acids or fatty alcohol ethoxylates containing 12 to 22 carbon atoms in the fatty group) and 1 to 50% by weight of surfactants with an HLB value of preferably 4 to 20. International patent application WO 95/35412 discloses similar tissue papers where water-free mixtures of (a) mineral oils, (b) fatty alcohols or fatty acids and (c) fatty alcohol ethoxylates are used as softeners. International patent application WO 95/16824 describes softening compositions for tissue papers containing mineral oil, fatty alcohol ethoxylates and nonionic surfactants (sorbitan esters, glucamides). In addition, International patent application WO 97/30216 (Kaysersberg) describes softening compositions for paper handkerchiefs which contain (a) 35 to 90% by weight of long-chain fatty alcohols, (b) 1 to 50% by weight of wax esters containing 24 to 48 carbon atoms, (c) 0 to 20% by weight of nonionic emulsifiers and (d) 0 to 50% by weight of mineral oil. WO 02/056841 (Cognis) describes emulsions comprising polyolpoly-12-hydroxystearate, alkylpolyglucosides, oil and 5 to 30 % of water. Although the emulsion described in this WO reference shows a good stability under usual conditions, stability problems may occur if it is stored for very long times or at higher temperatures. Further, it was noted that the incorporation of plant extracts into this emulsion causes colouring which lowers whiteness (brightness) of tissue paper treated with this lotion. It was therefore an object of the invention to provide emulsions, which can contain coloured ingredient, e.g. plant extracts, but display a high degree of whiteness when applied to a substrate, such as e.g. paper or textiles. Moreover, improvements in softness also seem to be possible.

Another object of the invention was to provide emulsions which display excellent care properties, would resemble conventional skin-care formulations in their sensory properties and would be distinguished by particular mildness and dermatological compatibility.

Another object of the invention was to provide emulsions which display a low odour and maintain this low odour upon usage on substrates, especially under long term storage conditions. A further object of the invention is directed to the capacity of the emulsion to contain perfumes and to maintain the perfume characteristics upon usage on a substrate. These properties are especially relevant for the usage of the emulsions in end-consumer products, in which the "smell" or odour is highly relevant for the customers acceptance of the product.

In a further object of the invention, it is desired that the emulsion displays a rheological profile, which makes it possible to apply the emulsion in cosmetic composition or on a substrate at comparatively low temperatures. This is desired not only with respect to temperature sensitive ingredients (colour, smell, stability) but also to economically considerations of large scale production sites (cost factor)

In view of the above, it is one object of the present invention to provide an emulsion that overcomes disadvantages of prior art formulations.

It is a further object of the present invention to provide an emulsion which displays a suitable, particular improved balance of critical properties including stability, softness and/or sensory impression, compatibility with plant extracts and application temperature.

Accordingly, the problem addressed by the present invention was to provide emulsions, which when applied to utility papers, more particularly tissue papers, and tissue cloths show a particularly agreeable soft feel in the final product. The emulsions would have excellent care properties, would resemble conventional skin-care formulations in their sensory properties and would be distinguished by particular mildness and dermatological compatibility. Another aspect of the problem addressed by the invention was to provide emulsions which can be used to on tissue papers which have a large recycled paper content. At the same time, only readily biodegradable auxiliaries would be used and the emulsions would penetrate easily into the tissue, would be uniformly dispersed therein and, even in highly concentrated form, would have such a low viscosity that they would be easy to process and would allow the coated tissues to sink and dissolve in water.

### SUMMARY OF THE INVENTION

It has now been found that emulsions based on a certain emulsifier combination and oil components with a defined water content significantly improve the softness of the paper products, have excellent sensory properties, are easy to process, even in the case of particularly critical tissue paper with a large recycled paper content, and are distinguished by particular mildness. In addition these emulsions display an advantageous rheological profile, display a high degree of whiteness when applied to a substrate - even if they contain coloured ingredients, such as e.g. plant extracts. In addition these emulsions display an advantageous odour profile, that is they maintain their low odour upon application on a substrate, e.g. paper.

The term "containing" as used throughout the invention is identical to the term "comprising" and is meant to be cover the more limiting expression "essentially consisting of and "consisting of".

In one embodiment the invention is related to O/W emulsions contains
- at least one non-ionic emulsifier
- at least one anionic co-emulsifier
- one oil component having a polarity of at least 20 mN/m or a mixture of oil components wherein at least 75 weight-% of the oils constituting the mixture have a polarity of at least 20 mN/m
- 6 to 35 weight-% of water based on the total weight of the emulsion

Wherein the total the total amount of emulsifier(s) is between 4 and 20 weight-%, preferably between 6 and 16 weight-%, based on the total weight of the emulsion.

In a preferred embodiment of the invention the O/W emulsion contains
(a) at least one non-ionic emulsifier having an HLB value of less than 10, preferably ≤ 8, more preferably ≤ 5, e.g. 2 to 5
(b) at least one further non-ionic emulsifier having an HLB value of more than 10, preferably ≥ 12, preferably from 12 to 20 (e.g. 15 to 18)
(c) at least one anionic co-emulsifier
(d) one oil component having a polarity of at least 20 mN/m or a mixture of oil components wherein at least 75 weight-% of the oils constituting the mixture have a polarity of at least 20 mN/m
(e) 6 to 35 weight %, preferably 10 to 30 weight % of water based on the total weight of the emulsion
(f) optionally at least one consistency regulator, and
(g) optionally at least one humectant.

In a preferred embodiment of the invention the O/W emulsion contains as emulsifier (a) at least one polyol polyester wherein the polyhydric alcohol having at least two hydroxyl groups is esterified with at least one acid having from 6 to 30 carbon atoms ant ad least one hydroxyl group or condensations product(s) of this hydroxyl fatty acid.

Accordingly, the present invention relates to O/W emulsions containing:
(a) at least one polyol poly-12-hydroxystearate
(b) at least one further non-ionic emulsifier
(c) at least one anionic co-emulsifier
(d) oil, where at least 75 weight-% of the oil has a polarity of ≥ 20 mN/m
(e) 10 to 30 weight -% water based on the total weight of the emulsion
(f) optionally at least one consistency regulator
(g) optionally at least one humectant
- whereas the total amount of emulsifiers [=(a) plus (b) plus (c)] is between 4 and 20 weight-%, preferably between 6 and 16 weight-%, based on the total weight of the emulsion.

It preferably consists essentially of the above components and up to 15 weight % other ingredients, such as for example consistency regulators, humectants, cosmetic agents and/or further components.

Unless otherwise specified, "weight %" always refers to "weight % based on the total weight of the emulsion". The term "emulsifier" as used throughout the description always relates to one or more single emulsifier as well as to mixtures of emulsifiers.

It is preferred that a tissue paper treated with this emulsion, even if containing a plant extract, shows a degree of whiteness (brightness) that is improved by about 4% when compared to a tissue paper treated with a lotion as disclosed in WO 02/057547. The whiteness was measured according to DIN EN 12625-7, item 7.3.2, colour (D65/10°), using a Minolta Spectrometer CM-3610d, the whiteness of tissue paper without the emulsion being 100 %.

Surprisingly, it was found that the emulsion according to the present invention displays a viscosity minimum at preferred low application temperatures ranging from 25 to 45°C, in particular 30 to 40°C, e.g. 32 to 38°C.
In addition, is was surprisingly found that the viscosity of the emulsion is stable in the temperature range of 32 to 38 °C,

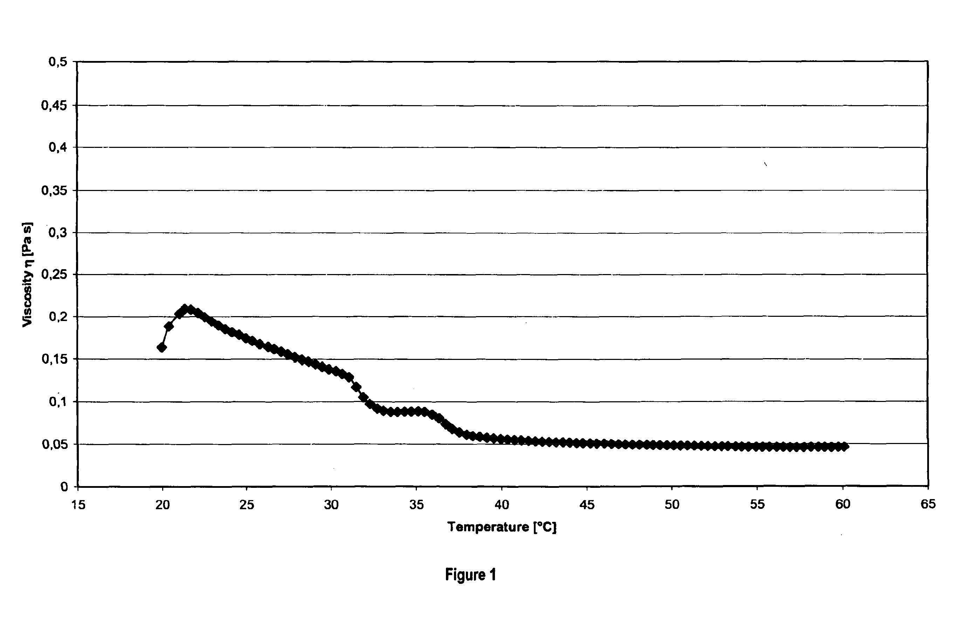

If measured by a rheometer, the emulsion used in the present invention shows a viscosity [mPas] of less than 1, preferably less than 0,8, in particular less than 0,5 (e.g. 0,01 to 0,3) in the temperature range of 20 to 60 °C (shear rate D= 50 I/sec, CR mode (rotation), constant heating from 20 to 60°C over 450 sec, number of measuring points 200), as shown in fig.1. The lotion measured is described in detail in example 1 of this description.
The measurement was conducted with a Haake RheoStress RS1 Rheometer (now available from Thermo Electron) under the following additional conditions regarding measurement geometry: sensor C35/2° Ti, A-factor of 89090.000 Pa/Nm, M-factor of 28.650 (I/s)/(rad/s), moment of inertia: 1.769e-06 kg m², attenuation of 30.00 and slit width 0.105 mm. The tempering device used was TCP/P (Pelletier/Plate).

The emulsion can be a semi-solid or a viscous liquid at room temperature (23°C), the latter being preferred. In case the emulsion is semi-solid, it typically has a viscosity of less than 30000 mPas at 25°C (measured with a Brookfield-RVF Viscosimeter, spindle 3, 10 rpm).

The viscosity of the emulsion can be adjusted, as known in the art, by the use of higher or lower amounts of solid components, in particular consistency regulators mentioned below. Further the homogenization of the emulsions (energy influx) may have an impact on the final viscosity. The melting range of the optionally present solid components, as measured according to DSC analysis of the final emulsion composition, preferably lies within the temperature range of 25 to 70 °C, in particular 30 °C to 60°C.

Moreover, the emulsion as such displays a soft and agreeable sensory impression either when used directly for a cosmetic composition or when applied to a substrate such as e.g. paper. In addition the emulsion is capable of transferring active agents to the skin of the user, if necessary, either directly from the cosmetic composition or from the substrate onto which is has been applied.

### DETAILED DESCRIPTION OF THE INVENTION

In a preferred embodiment the emulsions have a viscosity of 100 to 10,000 mPa•s at 25°C, preferably of 500 to 4,000, more preferably of 2,000 to 3,000 measured according to Brookfield RVF, spindle 3, 10 rpm. Accordingly the emulsions can be easily processed, even in concentrated form.

### Component (a) non-ionic emulsifier

The emulsifier (a), which can be a single emulsifier or a mixture of emulsifiers is of a non-ionic type and primarily has the function of forming an oil-in-water emulsion. It can be suitably selected from know non-ionic O/W or W/O emulsifiers or combinations thereof.

The emulsifier (a) can be selected from the group consisting of hydrophilic O/W emulsifiers. These hydrophilic emulsifiers ay have an HLB value of 10 to 20. Such emulsifiers are known from the prior art and some are, for instance, listed in Kirk-Othmer, Encylopedia of Chemical Technology, third edition 1979, volume 8, page 913. According to the invention, the HLB value for ethoxylated products may also be calculated according to the formula: HLB = (100-L) : 5, wherein L is the weight percentage of hydrophilic groups, e.g. fatty alkyl or fatty acyl groups present in the ethoxylated products.

Preferably a liquid O/W emulsifier is used, although the use of minor amounts of solid emulsifiers is possible depending on the desired viscosity of the resulting emulsion.

Also W/O emulsifiers are suitable as emulsifiers (a) according to the present invention. W/O emulsifiers show a lower HLB value as the O/W emulsifiers. Typically W/O emulsifiers have HLB values below 10, preferably ≤ 8, more preferably ≤ 5, e.g. 2 to 5.

Component (a) preferably respresents a liquid polyol polyester wherein a polyol having at least two hydroxyl groups is esterified with at least one carboxylic acid having from 6 to 30 carbon atoms (in particular 16 to 22 C atoms) and having at least one hydroxyl group or condenstioan products of this hydroxyl fatty acid. Polyols include monosaccharides, disaccarides, and trisaccharides, sugar alcohols, other sugar derivatives, glycerol, and polyglycerols, e.g. diglycerol, trigrlycerol, and higher glycerols. Such polyol preferably has from 3 to 12, in particular 3 to 8 hydroxy groups and 2 to 12 carbon atoms (on average, it if it a mixture as in polyglycerols). The polyol preferably is polyglycerol, in particular that having the specific oligomer distribution described in WO 95/34528 (page 5).

The carboxylic acid used in the polyol polyester preferably is a fatty acid ester having from 6 to 30 carbon atoms (hereinafter, unless stated otherwise, the term "fatty acid" is not limited to the naturally occurring, even-numbered, saturated or unsaturated long-chain carboxylic acids, but also includes their even-numbered homologues or branched derivatives thereof). The fatty acid contains at least one hydroxyl group. It can be a mixture of hydroxy fatty acids or a condensation product thereof (poly(hydroxy fatty acids)). The preferred carbon range for the above mentioned hydroxy fatty acids is from 16 to 22, in particular 16 to 18. A particularly preferred poly (hydroxy fatty acid) is the condensation product of hydroxyl stearic acid, in particular 12-hydroxy stearic acid, optionally in admixture with poly (ricinoleic acid), said condensation product preferably having the properties described in WO 95/34528.

In a preferred embodiment of the invention component (a) is a polyol poly-12-hydroxystearate.

### Component (a) polyol poly-12-hydroxystearate

The polyol poly-12-hydroxystearate which form component (a) are known substances which are marketed by Cognis Deutschland GmbH, for example under the names of "Dehymuls^{®} PGPH" and "Eumulgin^{®} VL 75" (mixture with Coco Glucosides in a ratio by weight of 1:1) or Dehymuls^{®} SBL. Particular reference is made in this connection to European Patent EP 0 766 661 B1. The polyol component of these emulsifiers may be derived from substances which contain at least 2, preferably 3 to 12 and more preferably 3 to 8 hydroxyl groups and 2 to 12 carbon atoms. Typical examples are
(a) glycerol and polyglycerol;
(b) alkylene glycols such as, for example, ethylene glycol, diethylene glycol, propylene glycol;
(c) methylol compounds such as, in particular, trimethylol ethane, trimethylol propane, trimethylol butane, pentaerythritol and dipentaerythritol;
(d) alkyl oligoglucosides containing 1 to 22, preferably 1 to 8 and more preferably 1 to 4 carbon atoms in the alkyl group such as, for example, methyl and butyl glucoside;
(e) sugar alcohols containing 5 to 12 carbon atoms such as, for example, sorbitol or mannitol,
(f) sugars containing 5 to 12 carbon atoms such as, for example, glucose or sucrose;
(g) amino sugars such as, for example, glucamine.

Among the emulsifiers suitable for use in accordance with the invention, reaction products based on polyglycerol are particularly important by virtue of their excellent application properties. Thus in a preferred embodiment the polyol-poly-12-hydroxystearate is a polyglycerol-poly-12-hydroxystearate. It has proved to be of particular advantage to use reaction products of poly-12-hydroxystearic acid with polyglycerols which have the following homolog distribution (the preferred ranges are shown in brackets):

| | |
|---|---|
| glycerol: | 5 to 35 (15 to 30) % by weight |
| diglycerols: | 15 to 40 (20 to 32) % by weight |
| triglycerols: | 10 to 35 (15 to 25) % by weight |
| tetraglycerols: | 5 to 20 (8 to 15) % by weight |
| pentaglycerols: | 2 to 10 (3 to 8) % by weight |
| oligoglycerols: | to 100 % by weight |

According to the invention, it is of particular advantage to use Eumulgin^{®} VL 75, an emulsifier mixture based on polyglycerol poly-12-hydroxystearate, lauryl glucosides and glycerol (ratio by weight 1:1:0.75) marketed by Cognis Deutschland GmbH.

In a preferred embodiment of the invention, the HLB value of polyol poly-12-hydroxystearate (a) is ≤ 10, preferably ≤ 8, more preferably ≤ 5.

In a preferred embodiment of the invention the polyol poly-12-hydroxystearate is present in the emulsions in a quantity of 2 to 10, preferably 3 to 7% by weight.

### Component (b) further non-ionic emulsifier

Possible further non-ionic emulsifiers which form component (b) are:
(1) products of the addition of 2 to 50 mol ethylene oxide and/or 0 to 20 mol propylene oxide onto linear fatty alcohols containing 8 to 40 carbon atoms, onto fatty acids containing 12 to 40 carbon atoms and onto alkylphenols containing 8 to 15 carbon atoms in the alkyl group;
(2) C_{12/18} fatty acid monoesters and diesters of addition products of 1 to 50 mol ethylene oxide onto glycerol;
(3) glycerol mono- and diesters and sorbitan mono- and diesters of saturated and unsaturated fatty acids containing 6 to 22 carbon atoms and ethylene oxide addition products thereof;
(4) alkyl mono- and oligoglycosides containing 8 to 22 carbon atoms in the alkyl group and ethoxylated analogs thereof;
(5) addition products of 7 to 60 mol ethylene oxide onto castor oil and/or hydrogenated castor oil;
(6) other polyol esters and, in particular, polyglycerol esters other than polyol poly-12-hydroxystearates such as, for example, polyglycerol polyricinoleate or polyglycerol dimerate.
(7) addition products of 2 to 60 mol ethylene oxide onto castor oil and/or hydrogenated castor oil;
(8) partial esters based on linear, branched, unsaturated or saturated C₆₋₂₂ fatty acids, ricinoleic acid and 12-hydroxystearic acid and glycerol, polyglycerol, pentaerythritol, dipentaerythritol, sugar alcohols (for example sorbitol), alkyl and/or alkenyl glucosides (for example methyl glucoside, butyl glucoside, lauryl glucoside) and polyglucosides (for example cellulose);
(9) wool wax alcohols;
(10) polysiloxane/polyalkyl/polyether copolymers and corresponding derivatives;
(11) mixed esters of pentaerythritol, fatty acids, citric acid and fatty alcohol and/or mixed esters of fatty acids containing 6 to 22 carbon atoms, methyl glucose and polyols, preferably glycerol or polyglycerol, and
(12) polyalkylene glycols.

The addition products of ethylene oxide and/or propylene oxide onto fatty alcohols, fatty acids, alkylphenols, glycerol monoesters and diesters and sorbitan monoesters and diesters of fatty acids or onto castor oil are known commercially available products. They are homolog mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide with glycerol are known as lipid layer enhancers for cosmetic preparations from DE 20 24 051.

The invention encompasses the use of several further non-ionic emulsifiers from one class as well as the use of mixtures of one or more further non-ionic emulsifiers from different classes. Since the emulsions according to the invention are O/W emulsions, it is particularly preferred to use non-ionic emulsifiers from the group of hydrophilic O/W emulsifiers.
In a preferred embodiment of the invention the HLB value of the further non-ionic emulsifiers (b) is ≥ 10, preferably ≥ 12, preferably between 12 and 20.

In principle, hydrophilic co-emulsifiers are emulsifiers with an HLB value of 10 to 20 which are listed in numerous tables and are well-known to the expert. Some of these emulsifiers are listed, for example, in Kirk-Othmer, "Encyclopedia of Chemical Technology", 3rd Edition, 1979, Vol. 8, page 913**.** According to the invention, the HLB value for ethoxylated products may also be calculated to the following formula: HLB = (100-L) : 5, where L is the percentage by weight of lipophilic groups, i.e. fatty alkyl or fatty acyl groups, in percent by weight in the ethylene oxide adducts.

In a preferred embodiment according to the invention the further non-ionic emulsifier (b) is selected from the group consisting of
- (b-1) products of the addition of 2 to 50 mol ethylene oxide and/or 0 to 20 mol propylene oxide onto linear fatty alcohols containing 8 to 40 carbon atoms, onto fatty acids containing 12 to 40 carbon atoms and onto alkylphenols containing 8 to 15 carbon atoms in the alkyl group
- (b-2) C_{12/18} fatty acid monoesters and diesters of addition products of 1 to 50 mol ethylene oxide onto glycerol;
- (b-3) glycerol mono- and diesters and sorbitan mono- and diesters of saturated and unsaturated fatty acids containing 6 to 22 carbon atoms and ethylene oxide addition products thereof;
- (b-4) alkyl and/or alkenyl polyglycosides, preferably alkyl and/or alkenyl polyglycosides containing 6 to 22 carbon atoms in the alkyl group and ethoxylated analogs thereof;
- (b-5) partial esters based on linear, branched, unsaturated or saturated C₆₋₂₂ fatty acids, ricinoleic acid and 12-hydroxystearic acid and glycerol, polyglycerol, pentaerythritol, -dipentaerythritol, sugar alcohols, alkyl glucosides and polyglucosides; or
- (b-6) polysiloxane/polyalkyl/polyether copolymers and corresponding derivatives;
- and mixtures thereof

Of particular advantage from the group of O/W emulsifiers are, for example, Ceteareth-12, Ceteareth-20, PEG-30 Stearate, PEG-20 Glyceryl Stearate, PEG-40 Hydrogenated Castor Oil and Polysorbate 20.

In a preferred embodiment of the invention the further non-ionic emulsifier (b) selected from groups (b-4) and (b-5).

In a preferred embodiment the further non-ionic emulsifier (b) is at least one an alkyl and/or alkenylpolyglycoside corresponding to the general formula RO-(Z)p. These alkyl and/or alkenyl polyglycosides support the mildness of the compositions according to the invention. In particular they support the formation of O/W emulsions despite of the low water content.

They may be obtained by the relevant methods of preparative organic chemistry.
EP-A1-0 301 298 and WO 90/03977 are cited as representative of the extensive literature available on this subject.

The preferred alkyl and/or alkenyl polyglycosides are characterized by the following parameters: The alkyl and/or alkenyl group R contains 4 to 22, preferably 6 to 22 carbon atoms and may be both linear and branched. Primary, linear or 2-methyl-branched aliphatic groups are preferred. Alkyl groups such as these are, for example, 1-octyl, 1-decyl, 1-lauryl, 1-myristyl, 1-cetyl and 1-stearyl. Particularly preferred are 1-octyl, 1-decy, 1-lauryl and 1-myristyl. Where so-called "oxo alcohols" are used as starting materials, compounds with an odd number of carbon atoms are dominant in the alkyl chain. The alkyl and/or alkenyl polyglycosides usable in accordance with the invention may, for example, contain only a certain alkyl and/or alkenyl group R. However, these compounds are normally prepared from natural fats and oils or mineral oils. In this case, the alkyl and/or alkenyl groups R are mixtures corresponding to the starting compounds or to the particular method used for working up these compounds.

The alkyl or alkenyl radical R may be derived from primary alcohols containing 4 to 11 and preferably 8 to 10 carbon atoms. Typical examples are butanol, caproic alcohol, caprylic alcohol, capric alcohol and undecyl alcohol and the technical mixtures thereof obtained, for example, in the hydrogenation of technical fatty acid methyl esters or in the hydrogenation of aldehydes from Roelen's oxosynthesis. Alkyl polyglycosides having a chain length of C 8 to C 10 (DP=1 to 3), which are obtained as first runnings in the separation of technical C 8-18 coconut oil fatty alcohol by distillation and which may contain less than 6% by weight of C 12 alcohol as an impurity, and also alkyl polyglycosides based on technical C 9/11 oxoalcohols (DP=1 to 3) are preferred. In addition, the alkyl or alkenyl radical R may also be derived from primary alcohols containing 12 to 22 and preferably 12 to 14 carbon atoms. Typical examples are lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol, brassidyl alcohol and technical mixtures thereof which may be obtained as described above. Alkyl polyglycosides based on hydrogenated C2/14 coconut oil fatty alcohol having a DP of 1 to 3 are preferred.

In a preferred embodiment of the invention alkyl polyglycosides are used.

In particularly preferred alkyl polyglycosides, R consists
- essentially of C₈ and C₁₀ alkyl groups,
- essentially of C₁₂ and C₁₄ alkyl groups,
- essentially of C₁₆ and C₁₈ alkyl groups,
- essentially of C₈ to C₁₆ alkyl groups or
- essentially of C₁₂ to C₁₆ alkyl groups.

The sugar unit Z may be selected from mono- or oligosaccharides. Sugars containing 5 or 6 carbon atoms and the corresponding oligosaccharides are normally used. Examples of such sugars are glucose, fructose, galactose, arabinose, ribose, xylose, lyxose, allose, altrose, mannose, gulose, idose, talose and sucrose. Preferred sugar units are glucose, fructose, galactose, arabinose and sucrose. Glucose is particularly preferred.

The term "alkyl and/or alkenyl polyglycoside" as used throughout the description encompasses alkyl and/or alkenyl glycosides with one sugar unit (alkyl and/or alkenyl mono-glycosides; p = 1) as well as alkyl and/or alkenyl glycosides with two to five sugar units (alkyl and/or alkenyl oligo-glycosides, p = 2 to 5) as well as alkyl and/or alkenyl glycosides with more than 5 sugar units (alkyl and/or alkenyl polyglycosides), preferably up to 10 sugar units, as well as mixtures thereof.

The index p in general formula RO-(Z)p indicates the degree of polymerization (DP), i.e. the distribution of mono-, oligo and polyglycosides, and is preferably a number of 1 to 10. Whereas p in a given compound must always be an integer and, above all, may assume a value of 1 to 6, the value p for a certain alkyl and/or alkenyl oligoglycoside is an analytically determined calculated quantity which is generally a non whole number.

The alkyl and/or alkenyl polyglycosides preferably usable in accordance with the invention contain on average 1.1 to 5 sugar units. Alkyl and/or alkenyl polyglycosides with values for p of 1.1 to 3, preferably 1.1 to 1.7 are preferred. Alkyl and/or alkenyl polyglycosides in which p has a value of 1.2 to 1.5 and more particularly 1.4 to 1.5 are particular preferred.

In a particularly preferred embodiment, the further non-ionic emulsifier is a lauryl glucoside. These are C₁₂₋₁₆ fatty alcohol glucoside mixtures with an average degree of oligomerization (degree of polymerization) p of 1.4. Various fatty alcohol glucosides usable in accordance with the invention are marketed by Cognis Deutschland GmbH, for example under the name of Plantacare^{®}.

The alkoxylated homologs of the alkyl and/or alkenyl polyglycosides mentioned may also be used in accordance with the invention. These homologs may contain on average up to 10 ethylene oxide and/or propylene oxide units per alkyl and/or alkenyl glycoside unit.

In a preferred embodiment of the invention the further non-ionic emulsifier (b) is present in the emulsions in a quantity of 2 to 10, preferably 3 to 7% by weight.

In a preferred embodiment of the invention the weight ratio of polyol-poly-12-hydroxystearate (a) to further non-ionic emulsifier (b) is in the range of 1:0,5 to 1:2, preferably in the range of 1 : 0,7 to 1: 1,5, most preferably in the range of 1 : 0.8 to 1:1,2.

### Component (c) anionic co-emulsifier

Anionic co-emulsifiers which form component (c) are characterized by a water-solubilizing anionic group, such as for example a carboxylate, sulfate, sulfonate or phosphate group, and a lipophilic residue. Even though this is not always mentioned in the following, the anionic co-emulsifier must also comprise for reasons of charge neutrality a positive counter ion which is preferably selected from hydrogen, ammonium and alkali salts such as sodium or potassium. Accordingly the anionic co-emulsifier is employed in salt form.

Dermatological compatible anionic emulsifiers are known to the expert in large numbers from relevant manuals and are commercially available. More particularly, they are alkyl sulfates in the form of their alkali metal, ammonium or alkanolammonium salts, alkyl ether sulfates, alkyl ether carboxylates, acyl isethionates, acyl sarcosinates, acyl taurines with linear C₁₂₋₁₈ alkyl or acyl groups and sulfosuccinates and acyl glutamates in the form of their alkali metal or ammonium salts. In a preferred embodiment of the invention the alkali metal salts of fatty acids, alkyl sulphate and alkyl phosphates are used with an alkyl moiety of C 12 to C 22. Among the anionic surfactants, alkali metal salts of fatty acids (such as e.g. sodium stearate, alkali metal salts of palmitic acid or behenic acid) and, in particular, alkyl sulfates (Lanette^{®} E) and alkyl phosphates (Amphisol^{®} K) are particularly suitable according to the invention because they lead to particularly stable and homogeneous emulsions. The term "fatty acid" as used in this context, is not restricted to the naturally occurring even numbered saturated and unsaturated long chain carboxylic acids. It also comprises uneven numbered homologues, as well as branched or substituted derivatives thereof. It is preferred to use saturated linear fatty acids such as lauric acid, myristic acid, palmitic acid or stearic acid. The amino acid can be any naturally occurring amino acid or synthetic analogue thereof including for instance alanine, valine, leucine, isoleucine, glycerine, serine, threonine. The amino acid is preferably selected from dicarboxylic acids having from 3 to 8 carbon atoms and one amino function such as glutamic acid or asparginic acid.

In a preferred embodiment of the invention the anionic co-emulsifier (c) is selected from the group of phosphate-, sulphate and carboxylate emulsifiers.

Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

In an especially preferred embodiment of the invention the anionic co-emulsifier (d) is a carboxylate emulsifier, preferably an acyl glutamates or an acyl-asparaginate.

### Acyl glutamates as component (c)

Acyl glutamates are known anionic emulsifiers corresponding to the following formula 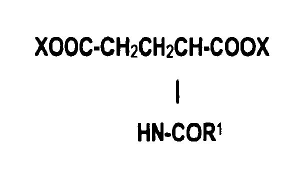 in which R¹CO is a linear or branched acyl radical containing 6 to 22 carbon atoms and 0 and/or 1, 2 or 3 double bonds and X is hydrogen, an alkali metal and/or alkaline earth metal, ammonium, alkylammonium, alkanolammonium (e.g. triethanolammonium) or glucammonium. They are produced, for example, by Schotten-Baumann acylation of glutamic acid with fatty acids, fatty acid esters or chlorides. Corresponding commercial products are available, for example, from Hoechst AG, Frankfurt, Germany or from the Ajinomoto Co. Inc., Tokyo, Japan. An overview of the production and properties of acyl glutamates was published by M. Takehara et al, in J. Am. Oil. Chem. Soc., 49, 143 (1972**).** Typical examples of suitable acyl glutamates suitable for the purposes of the invention are anionic surfactants derived from fatty acids containing 6 to 22 and preferably 12 to 18 carbon atoms, for example C 12/14 or C 12/18 cocofatty acid, lauric acid, myristic acid, palmitic acid and/or stearic acid. Sodium or potassium N-cocoyl and sodium or potassium N-stearoyl-L-glutamate are particularly preferred. Triethanolamine-salts of acyl glutamates are also preferred, especially the triethanolamine salt of N-cocoylglutatmate and the triethanolamine salt of N-stearoyl-L-glutamate.

The anionic co-emulsifier is preferably an acyl glutamate or acyl asparaginate wherein the acyl residue may also be derived from other carboxylic acids than the above mentioned fatty acids. More preferably the anionic co-emulsifier is an acyl glutamate, preferably a stearoyl glutamate such as the commercially available sodium stearoylglutamate.

In a preferred embodiment of the invention the **anionic co-emulsifier (c)** is present in an amount of 0,01 to 10, preferably 0,05 to 5 weight-%, preferably 0,03 to 1,4 weight-% based on the total weight of the emulsion.

In a preferred embodiment of the invention the **anionic co-emulsifier (c)** is present in an amount of 1 to 20 weight-% based on the amount of further non-ionic emulsifier (b).

It is to be understood that for this weight ratio, the total amount of anionic co-emulsifiers (c) and the total amount of further non-ionic emulsifiers (b) is to be considered.

The total amount of emulsifiers, that is component (a), component (b) and component (c), is between 4 and 20 weight-%, preferably between 6 and 16 weight-%, based on the total weight of the emulsion.

### Component (d) Oil

Hereinafter, the term "oil" is used for water-insoluble, organic, natural and synthetic, cosmetically useful oils having preferably a liquid (also viscous) consistency at room temperature (23°C). In the context of the invention, insolubility in water is understood to be a solubility of less than 10% by weight at 20°C.

The "oil" (d) used according to the invention can be a sole oil component or a mixture of more than one oil components.

The oil according to the invention is characterized in that at least 75 weight-% of the oil has a polarity of ≥ 20 mN/m.

The term "≥ " used throughout the description refers to "equal or greater than".

It is understood that the 75 weight-% are based on the total weight of the oil present in the emulsion. In a preferred embodiment at least 80, preferably at least 85, more preferably at least 90 or 95 weight-% of the oil has a polarity of ≥ 20 mN/m based on the total weight of the oil.

In a preferred embodiment at least 75, preferably at least 80, preferably at least 85, more preferably at least 90 or 95 weight-% of the oil has a polarity of ≥ 25 mN/m based on the total weight of the oil.

In a preferred embodiment at least 75, preferably at least 80, preferably at least 85, more preferably at least 90 or 95 weight-% of the oil has a polarity of ≥ 30 mN/m based on the total weight of the oil.

The polarity of the oil in combination with the choice of the emulsifiers has surprisingly led to emulsions which are superior as compared to those known in the art.

In a preferred embodiment of the invention at least 30 weight -%, preferably at least 40 weight -%, more preferably at least 50 weight-%, most preferably at least 60 weight-% of the oil (d) based on the total weight of the oil has a polarity of ≥ 35 mN/m.

In a preferred embodiment at least 75 weight-% of the oil has a polarity of ≥ 20 mN/m based on the total weight of the oil and at least 30 weight-% of the oil (d) based on the total weight of the oil has a polarity of ≥ 25 mN/m.
The polarity of an oil is defined by determining the polarity (= polarity index) of an oil against water. The following table lists the polarity for the most common oils.

Oils with a polarity of greater than 30 mN/m are considered as "non polar", oils with a polarity between 25 and 30 are mN/m are considered as "medium polar".

The following table lists the polarity for the most common oils

| **Common Oil (CTFA name)** | **Polarity index [mN/m]** |
|---|---|
| Non polar | |
| Isoparaffin (C 12-C14) | 53.0 |
| Squalan | 46.2 |
| Isohexadecan (ARLAMOL ND) | 43.8 |
| Mineral Oil (Paraffin oil perliquidum) | 43.7 |
| Mineral Oil (Paraffin oil subliquidum) | 38.3 |
| | |

| Polar | |
|---|---|
| Cetystearyloctanoate | 28.6 |
| Diemethicon (silicon oil 20 ct) | 26.6 |
| Isopropylpalmitate | 25.2 |
| Octyldodecanol | 24.8 |
| Dioctyladipate (ARLAMOL DOA) | 24.5 |
| Isopropymyristate | 24.2 |
| Octylpalmitate (2-ethylhexylpalmitate) | 23.1 |
| Hexamethyldisiloxan | 22.7 |
| Isopropylstearate | 21.9 |
| Carpyl/Caprine acid triglyceride (neutral oil) | 21.3 |
| Isopropylisostearate | 21.2 |
| Jojoba Oil | 20.8 |
| Cyclomethicone (ARLAMOL D4) | 20.6 |
| Peanut oil | 20.5 |
| Almond oil | 20.3 |
| Sunflower oil | 19.3 |
| Decyloleate | 18.7 |
| Avocado Oil | 18.3 |
| Olive oil | 16.9 |
| Castor oil | 13.7 |
| Calendula Oil | 11.1 |
| Wheat germ oil | 8.3 |

The polarity of oil can be determined using a ringtensiometer (e.G. Krüss K 10), measuring the boundary layer energy which is the boundary layer tension in mN/m. The lower limit is 5 mN/m. The method is suitable for low viscosity liquids given that a boundary layer is present (that is the liquids are not miscible). The polarity of the oil is determined against water.

The polarity of different oils is described for example in DE 102004003436 A1 on pages 8 to 11, which are hereby incorporated by reference.

The oil component (d) can be properly selected among known cosmetically useful oil. Such include **hydrocarbon-based oils**, such as aliphatic or aromatic oils. The hydrocarbon-based oil preferably has from 8 to 32, in particular 15 to 20 carbon atoms. Examples include squalane, squalene, paraffinic oils, isohexadecane, isoeicosane, polydecene or dialkycyclohexane, and mineral oil, mineral oil being preferred.

In a preferred embodiment of the invention the emulsion comprises a hydro-carbon based oil, preferably mineral oil as sole oil component or a mixture of (d-1) a hydrocarbon-based oil, preferably mineral oil and (d-2) at least one further oil.

Preferred **mineral oils** can be selected from white oil pharma 40 ("Weißöl Pharma 40"), fluid or liquid paraffin oil ("Paraffinöl dünnflüssig"), viscous paraffin oil ("Paraffinöl dickflüssig"), paraffinum liquidum, paraffinum perliquidum or paraffinum subliquidum.

Suitalbe as oil components are known oils other than hydrocarbon-based ones, e.g. oils from plant sources or synthetic oils. Examples of such oils are
- **Glycerides,** which are mono-, di- and/or tri ester (fatty acid ester) of glycerol (in particular di- and/or triester). Glycerides can be obtained by chemical synthesis or from natural sources (plant or animal) as known in the art. Preferably the fatty acid component has from 6 to 24, more preferably 6 to 18, in particular 8 to 18 carbon atoms. The fatty acid can be branched or unbranched as well as saturated or unsaturated. According to the invention the use of liquid glycerides from plant sources is preferred, in particular the use of a modified liquid coconut oil (INCl name: cocoglycerides, available under the trade name Myritol^{®} 331 from Cognis Deutschland GmbH) which contains as main component a mixture of di- and triglycerides based on C8 to C18 fatty acids.
- **Natural plant oils** which may contain liquid glycerides as main component, such as soja oil, peanut oil, olive oil, sunflower oil, macademia nut oil or jojoba oil.
- **Guerbet alcohols;** guerbet alcohols are based on fatty alcohols having 6 to 18, preferably 8 to 10 carbon atoms, such as 2-ethylhexanol or 2-octyldodecanol;
- **Fatty acid esters,** preferably those having 12 to 60 carbon atoms including
   a) esters of linear or branched, saturated or unsaturated C₆-C₂₄ fatty acids and linear or branched, saturated or unsaturated C₆-C₂₄ fatty alcohols (e.g. hexyl laurate, myristyl isostearate, myristyl oleate, cetyl isostearate, cetyl oleate, stearyl isostearate, stearyl oleate, isostearyl myristate, isostearyl palmitate, isostearyl stearate, isostearylisostearate, isostearyl oleate, hexyldecyl stearate, oleyl myristate, oleyl isostearate, oleyl oleate, oleyl erucate, erucyl isostearate, erucyl oleate, cococaprylatelcaprate).
   b) Esters of C₁₈-C₃₈ alkyl hydroxy carboxylic acids and linear or branched, saturated or unsaturated C₆-C₂₂ fatty alcohols,
   c) esters of linear and/or branched, saturated or unsaturated fatty acids and polyhydric alcohols (such as propylene glycol, dimerdiol or trimertriole) and/or guerbet alcohols, liquid triglycerides or triglyceride mixtures, or liquid mono-/di-triglyceride mixtures.
- **Esters from aromatic carboxylic acids,** such as esters of C₆-C₂₂ fatty alcohols and/or guerbet alcohols and aromatic carboxylic acids, in particular benzoic acid (e.g. Finsolv^{®}),
- **Esters of dicarboxylic acids,** in particular esters of C₂-C₁₂ dicarboxylic acids and linear or branched, saturated or unsaturated alcohols having 1 to 22 carbon atoms or polyols having 2 to 10 carbon atoms and 2 to 6 hydroxy groups.
- **Substituted cyclohexanes**
- Symmetric or asymmetric, linear or branched **dialk(en)ylethers** having from 6 to 24 carbon atoms (per alk(en)yl group, preferably having 12 to 24 C atoms as total number of C atoms), such as di-n-octylether (dicaprylylether), di-(2-ethylhexy)ether, laurylmethylether, octylbutylether or didocecylether, the use of di-n-octylether (dicaprylylether; viscosity: 2 - 5 mPaS at 20°C; DGF method described above) being preferred.
- **Dialk(en)ylcarbonates** having preferably at least one C6 to 22 alkyl or alkenyl group (preferred total number of C atoms: not more than 45, including the C atom of the carbonate unit). The alkyl or alkenyl group can be straight or branched. The alkenyl unit may display more than one double bond. They can be obtained by transesterification of dimethyl or diethyl carbonate in the presence of C6 to C22 fatty alcohols according to known methods (cf. Chem. Rev. 96, 951 (1996)). Typical examples for dialk(en)ylcarbonates are the (partial) transesterification products of caprone alcohol, capryl alcohol, 2-ethylhexanol, n-decanol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linolyl alcohol, linolenyl alcohol, elaeostearyl alcohol, arachidyl alcohol, gadoleyl alcohol, behenyl alcohol, erucyl alcohol and brassidyl alcohol as well as their technical mixture, which are for instance obtained by high pressure hydrogenisation of technical methyl ester(s) on fat or oil basis. Particularly suitable in view of their low viscosity at 20°C are dihexyl-, dioctyl-, di-(2-ethylhexyl)- or dioleylcarbonate (viscosity of dioctylcarbonate: 7 mPaS at 20°C; DGF method described above). Thus it is preferred to use either short chain (C6 to C10) alkyl or alkenyl carbonates.
- **Ring-opening products of epoxidized fatty acid esters and polyols,** and
- **silicon oils** of linear or cyclic structure such as dimethylpolysiloxane, methylphenylpolysiloxane, cyclomethicone, as well as amino-, fatty acid-, alcohol-, polyether-, epoxy-, fluorine-, glycosyde and/or alkyl-modified silicon oil. Further, dimethicone oils can be used. Cosmetically useful silicon oils are e.g. those of US 4,202,879 and US 5,069,897.

Generally, it is preferred to select the liquid embodiments among the above compound types.

In a preferred embodiment of the invention the oil (d) comprises at least one hydrocarbon-based oil, preferably mineral oil. In this embodiment of the invention it is especially preferred that the hydrocarbon-based oil (d) constitutes at least 40 weight-%, more preferably at least 50 weight-%, most preferably at least 60 weight-% of the oil (d), based on the total weight of the oil.

In a preferred embodiment of the invention the oil (d) comprises an oil which is selected from the group consisting of liquid synthetic triglyceride mixtures; vegetable oils; guerbet alcohols; liquid linear or branched carboxylic acid esters; liquid substituted cyclohexanes; symmetric or asymmetric dialk(en)ylethers having from 6 to 22 C atoms per alk(en)yl group; linear or branched dialk(en)ylcarbonates derived from C 6 to 22 fatty alcohols; ring-opening products of epoxidized fatty acid esters and polyols; silicone oils and mixtures thereof.

The liquid linear or branched carboxylic acid esters, preferably fatty acid esters are more preferably esters of monovalent carboxylic acids having at least one long chain alkyl or acyl residue (each having at least 6 C-atoms, in particular at least 12 C-atoms). Preferred carboxylic acid ester type emollient oils include those having more than 6 carbon atoms in total which comprise either an acyl or an alkyl residue having each 1 to 5 carbon atoms. According to even more preferred embodiments, the carboxylic acid ester has the following formula

R¹-COO-R²

wherein
i. R¹-CO represents an acyl residue having 6 to 28 carbon atoms, and R² represents an alkyl residue having 1 to 5 carbon atoms or
ii. R¹-CO represents an acyl residue having 1 to 5 carbon atoms and R² represents an alkyl residue having 6 to 28 carbon atoms.

In line with option (i) the acyl residue may be saturated or unsaturated (e.g. 1,2,3 double bonds), the saturated embodiments being preferred. The acyl residue, preferably the saturated acyl residue may be branched and is optionally substituted, although this is not preferred. Similarly the alkyl residue may be branched as in isopropyl and/or substituted. The acyl residue preferably has 12 to 22 carbon atoms, in particular 14 to 20 carbon atoms. The alkyl residue preferably has 1 to 3 carbon atoms as in methyl, ethyl, or (iso)propyl. Representative examples of such esters include methyl palmitate, methyl stearate, isopropyl laurate, isopropyl myristate and isopropyl palmitate.

In line with option (ii) the acyl residue may be branched and/or substituted for instance by hydroxyl. According to one embodiment of option (ii) the acyl residue has 2 to 4 carbon atoms. The alkyl residue preferably has 12 to 22 carbon atoms, in particular 14 to 20 carbon atoms. be saturated or unsaturated (e.g. 1,2,3 double bonds), the saturated embodiments being preferred. Moreoever the alky residue may also be branched and/or substituted. Suitable fatty acid ester emollients of type (ii) include lauryl lactate and cetyl lactate.

In a preferred embodiment the oil comprises a liquid linear or branched carboxylic acid ester, which comprises either an acyl or an alkyl residue having each 1 to 5 carbon atoms.

In a particularly preferred embodiment of the invention the oil (d) has a viscosity of 1 to 100 mPa•s, preferably ≤ 50 mPa•s; more preferably 1 to 20 mPa•s measured with a Höppler falling sphere viscosimeter at 20°C (method DGF C-IV 7).

In a preferred embodiment of the invention the emulsion comprises a mixture of (d-1) a hydrocarbon-based oil and (d-2) at least one oil component. In a preferred embodiment the further oil component (d-2) is chosen from the group consisting of liquid synthetic triglyceride mixtures; vegetable oils; guerbet alcohols; liquid linear or branched carboxylic acid esters; liquid substituted cyclohexanes; symmetric or asymmetric dialk(en)ylethers having from 6 to 22 C atoms per alk(en)yl group; linear or branched dialk(en)ylcarbonates derived from C 6 to 22 fatty alcohols; ring-opening products of epoxidized fatty acid esters and polyols; silicone oils and mixtures thereof.

In a preferred embodiment of the invention the emulsion comprises a mixture of (d-1) a hydrocarbon-based oil, preferably mineral oil and (d-2) at least one liquid linear or branched carboxylic acid ester. In this embodiment it is especially preferred that (d-2) is selected from the carboxylic acid ester of the following formula

R¹-COO-R²

wherein
(i) R¹-CO represents an acyl residue having 6 to 28 carbon atoms, and R² represents an alkyl residue having 1 to 5 carbon atoms or
(ii) R¹-CO represents an acyl residue having 1 to 5 carbon atoms and R² represents an alkyl residue having 6 to 28 carbon atoms.

Option (i) being more preferred.

The oil (d) is preferably present in an amount of 20 to 80, preferably 30 to 75, preferably 40 to 70, preferably 45 to 65 weight-% based on the total weight of the emulsion.

### Component (e) Water

The emulsions according to the invention contain 6 to 35 weight-% of water, preferably 12 to 32 weight-%, more preferably 10 to 30 weight-% based on the total amount of the emulsion.

The emulsions according to the invention contain 10 to 30 weight-% based on the total weight of the emulsion of water.

It can be of particular advantage in accordance with the invention to use water in a quantity of 20 to 30% by weight, preferably 21 to 29% by weight and more particularly 22 to 28% by weight.

### Optional component (f) Consistency regulator

Consistency regulators (=consistency factors) are substances which have a thickening, i.e. viscosity-increasing, effect in emulsions. The consistency factors are normally present in a quantity of 0.0 to 10% by weight, preferably in a quantity of 0.5 to 8% by weight and more particularly in a quantity of 2 to 7% by weight.

In a preferred embodiment of the invention the emulsion contains at least one consistency factor.

According to the invention, mono- and diglycerides or mono-diglyceride mixtures solid at 20°C, solid triglycerides and corresponding glyceride mixtures based on linear C₁₂₋₂₂ fatty acids and/or C₁₂₋₂₂ hydroxyfatty acids, fatty alcohols, waxes and soaps are particularly suitable.

According to the invention, mono-, di- or triglycerides solid at 20°C or mixtures thereof commercially available, for example, under the names of Cutina^{®} GMS, Syncrowax^{®} HGLC or Novata^{®} AB are particularly suitable as consistency factors. A glyceryl stearate marketed under the name of Cutina^{®} MD by Cognis Deutschland GmbH is particularly preferred.

According to the invention, other suitable consistency factors are, for example, preferably saturated C₁₂₋₂₄ fatty alcohols which are solid at 20°C. Such alcohols include, for example, myristyl alcohol, cetyl alcohol, stearyl alcohol, erucyl alcohol, ricinoleyl alcohol, arachidyl alcohol, behenyl alcohol, brassidyl alcohol and Guerbet alcohols thereof. According to the invention, other suitable consistency factors are fatty alcohol cuts obtained by reduction of naturally occurring fats and oils such as, for example, bovine tallow, peanut oil, colza oil, cottonseed oil, soya oil, sunflower oil, palm kernel oil, linseed oil, castor oil, com oil, rapeseed oil, sesame oil, cocoa butter and coconut oil. However, synthetic alcohols, for example the linear, even-numbered fatty alcohols from Ziegler's synthesis (Alfols^{®}) or the partly branched alcohols from the oxosynthesis (Dobanols^{®}) may also be used. The mixture of C₁₆/C₁₈ fatty alcohols marketed by Cognis Deutschland under the name of Lanette^{®} O is particularly suitable for the purposes of the invention.

Waxes are understood to be natural or synthetic materials with the following properties: they are solid or fragile and hard in consistency, coarsely to finely crystalline, transparent or opaque, but not glass-like, and melt above 35°C without decomposing. They are low in viscosity and non-stringing only slightly above their melting point and show highly temperature-dependent consistency and solubility. Waxes suitable for use in accordance with the present invention are, for example, natural vegetable waxes such as, for example, candelilla wax, carnauba wax, Japan wax, espartograss wax, cork wax, guaruma wax, rice oil wax, sugar cane wax, ouricury wax, montan wax, sunflower wax, fruit waxes, such as orange waxes, lemon waxes, grapefruit wax, bayberry wax, and animal waxes such as, for example, beeswax, shellac wax, spermaceti, wool wax and uropygial fat. According to the invention, it can be of advantage to use hydrogenated waxes. Natural waxes usable in accordance with the invention also include the mineral waxes, such as ceresine and ozocerite for example, or the petrochemical waxes, for example petrolatum, paraffin waxes and microwaxes. Other suitable wax components are chemically modified waxes, more particularly the hard waxes such as, for example, montan ester waxes, sasol waxes and hydrogenated jojoba waxes. Synthetic waxes usable in accordance with the invention include, for example, wax-like polyalkylene waxes and polyethylene glycol waxes, silicone waxes or esters of long-chain carboxylic acids with long-chain fatty alcohols. According to the invention, beeswax is preferably used as a consistency factor.

In the context of the invention, soaps are understood to be salts of fatty acids. Suitable consistency factors are, for example, alkali metal and alkaline earth metal and aluminium salts of C₁₂₋₂₄ fatty acids or C₁₂₋₂₄ hydroxyfatty acids, calcium, magnesium or aluminium stearate being preferred.

### Optional ingredient (g) humectant

In another preferred embodiment, the emulsion according to the invention contains at least one humectant. The humectant contributes towards improving the sensory properties and the stability of the emulsion and serves to regulate the skin moisture level. The humectants are normally present in a quantity of 1 to 20% by weight, preferably 5 to 15% by weight and more particularly 5 to 10% by weight.

According to the invention, suitable humectants are inter alia amino acids, pyrrolidone carboxylic acid, lactic acid and salts thereof, lactitol, urea and urea derivatives, uric acid, glucosamine, creatinine, cleavage products of collagen, chitosan or chitosan salts/derivatives and, in particular, polyols and polyol derivatives (for example glycerol, diglycerol, triglycerol, ethylene glycol, propylene glycol, butylene glycol, erythritol, 1,2,6-hexanetriol, polyethylene glycols, such as PEG-4, PEG-6, PEG-7, PEG-8, PEG-9, PEG-10, PEG-12, PEG-14, PEG-16, PEG-18, PEG-20), sugars and sugar derivatives (inter alia fructose, glucose, maltose, maltitol, mannitol, inositol, sorbitol, sorbityl silanediol, sucrose, trehalose, xylose, xylitol, glucuronic acid and salts thereof), ethoxylated sorbitol (Sorbeth-6, Sorbeth-20, Sorbeth-30, Sorbeth-40), honey and hydrogenated honey, hydrogenated starch hydrolyzates and mixtures of hydrogenated wheat protein and PEG-20-acetate copolymer. According to the invention, particularly preferred humectants are glycerol, diglycerol and triglycerol.

### Optional ingredient (h) cosmetic agent

A preferred embodiment of the emulsion according to the invention contains at least one cosmetic agent, preferably at least one irritation-soothing/anti-inflammatory agent which is intended in particular to soothe inflammatory skin processes or reddened, sore skin. The cosmetic agent is normally present in a quantity of 0.01 to 10% by weight, preferably 0.1 to 7% by weight and more particularly 1 to 5% by weight.

According to the invention, bisabolol, allantoin and panthenol and bisabolol are particularly preferred. Vitamins and vitamin precursors and protein hydrolyzates can also promote wound healing.

Also suitable are plant extracts which often contain a synergistic combination of wound-healing/irritation-soothing substances. These extracts are normally obtained by extraction of the whole plant. In individual cases, however, it can also be preferred to prepare the extracts exclusively from flowers and/or leaves of the plant.

As far as the plant extracts suitable for use in accordance with the invention are concerned, reference is made in particular to the extracts listed in the Table beginning on page 44 of the of the **3rd Edition of the Leitfaden zur Inhaltsstoffdeklaration kosmetischer Mittel,** published by the Industrieverband Körperpflege- und Waschmittel e.V. (IKW), Frankfurt.

According to the invention, the extracts of, above all, camomile, aloe vera, hamamelis, lime blossom, horse chestnut, green tea, oak bark, stinging nettle, hops, burdock root, horse willow, hawthorn, almond, pine needle, sandalwood, juniper, coconut, mango, apricot, lemon, wheat, kiwi, melon, orange, grapefruit, sage, rosemary, birch, mallow, lady's smock, creeping thyme, yarrow, thyme, balm, restharrow, coltsfoot, hibiscus, meristem, ginseng and ginger root are suitable.

One preferred type of cosmetic agents are plant extracts of the type as already mentioned above which often contain one or more wound-healing/soothing agents. Typically these extracts are prepared by extracting the entire plant. In some cases it can also be preferred to use solely the blossom and/or leaves of the plant. Preferred extracts are obtained from chamomile aloe vera, hamamelis, lime-blossom, sage and melissa. It is a particular merit of the present invention that plant extracts can be used without undesired colouring reactions of the emulsion.

Suitable extraction agents for the preparation of the plant extracts to be mentioned are water, alcohols and mixtures thereof. Among the alcohols, lower alcohols, such as ethanol and isopropanol, but especially polyhydric alcohols, such as ethylene glycol and propylene glycol, are preferably used both as sole extractant and in the form of mixtures with water. Plant extracts based on water/propylene glycol in a ratio of 1:10 to 10:1 have proved to be particularly suitable.

### Further components (i)

It is understood that the emulsion may contain further components usually present in emulsions, such as
- Preservatives which stabilize the emulsion, such as methylisothiazolin(on) which may have a chlorine as substituent, e.g. 5-chloro-2-methyl-4-isothiazolin-3-on or 2-methyl-4-isothiazolin-3-on; phenoxyethanol or PHB ester, paraben preservatives, pentanediol, sorbic acid or other compounds as mentioned in *"Kosmetikverordnung* (Cosmetics Regulation), Anlage 4, Teil A und B".
- Germicidal agent(s), e.g. those described in DE-199 06 081 A.
- Perfume, e.g. those described in DE 199 06 081; and/or
- Cosmetically useful dyes and pigments, e.g. those described in ***"***Kosmetische Färbemittel" (Cosmetic coloring agents), Verlag Chemie, Weinheim, 1984, p. 81-106", published by the "Farbstoffkommission der Deutschen Farbstoffgemeinschaft**".**

In a preferred embodiment the optional ingredients, like humectants (g), consistency factors (f), cosmetic agents (h) and further components (i) are present in an amount up to 15 % by weight, preferably in an amount of 0,01 to 10 % by weight.

### Preferred emulsion

The most preferred emulsion, which based on current knowledge reflects the best mode for carrying out the invention, comprises the following components:
O/W emulsion, characterized in that it contains
i) 3 to 7 weight-% of **polyol-12-poly-hydroxystearate (a),** preferably polyglycerol-12-poly-hydroxystearate
ii) 3 to 7 weight-% of a further **non-ionic emulsifier (b)**, preferably at least one alkyl and/or alkenyl polyglycoside
iii) 0.1 to 10 weight-% of at least **one anionic co-emulsifier (c)**
iv) 40 to 70 weight-% of an **oil component (d),** whereas at least 75 weight-% of the oil, has a polarity of ≥ 20 mN/m;
v) 10 to 30 weight-%, preferably 20 to 25 weight % of **water (e)**
vi) 0-5 weight-% of at least one **consistency regulator (f)**
vii) 0 -5 weight % of at least one humectant (g)
viii) 0-5 weight % of at least one cosmetic agent (h)
- whereas the total amount of emulsifiers [=(a) plus (b) plus (c)] is between 6,1 and 16 weight-%, based on the total weight of the emulsion

### Preparation of the emulsion

The emulsion (water-in-oil emulsion) can be prepared according to known methods.

One procedure involves mixing and homogeneously stirring the oil (d) and the emulsifiers (a), (b) and (c) and other optional oil-soluble additives at room temperature (usually for approximately 10 min). These components are typically highly soluble and give rise to a homogeneous mixture. The components of the water phase such as water, humectant, and possible water-soluble or water-dispersible additives such as perfume or preservatives are separately mixed at room temperature and slowly added to the mixture of oil-phase components during continuous stirring. After continued stirring (preferably for approximately 10 min) the resulting mixture is then homogenized (usually for approximately 10 min) with a suitable dispersion device such as supraton or stator-rotor homogenizers of Ultraturrax type (see for instance Karlheinz Schrader, Grundlagen und Rezepturen der Kosmetika, Hüthig Buch Verlag Heidelberg, Second Edition, 1989, pages 906 to 912**)**. As known from the prior art, homogenizing conditions may have an impact on the viscosity of the emulsion obtained. If the viscosity is too high, which is undesirable in the present invention, it is possible for instance to reduce the energy influx during homogenization, in particular by lowering the rotational speed of the rotor/stator system.

Further, it is possible to prepare the emulsion of the invention by mixing the oil phase and water phase components at a higher temperature. For this purpose, it is preferred to heat the oil phase and water phase components separately to about 80°C to 85°C. Then, at this temperature the water phase components are slowly added to the oil phase components while stirring, optionally homogenizing. After continued stirring, preferably for about 5 min, the mixture is allowed to cool while stirring in such a way that it remains in continuous motion. Simultaneously, the incorporation of air should be avoided. The mixture is then homogenized with a suitable dispersion device such as supraton or stator-rotor homogenizers of Ultraturrax type, preferably at 60° to 65° C, in order to improve stability and structure. After a homogeneous state is reached, the composition is allowed to cool to room temperature.

### Use of the emulsion

The emulsion according to the invention can suitably be used for the preparation of a cosmetic composition.

The emulsion according to the invention is especially suited for the application on a substrate. The substrate can be a fibrous web, such as a non-woven or tissue paper.

The emulsions according to the invention are especially suited for the application on tissue products such as handkerchiefs, personal care wipes such as cosmetic wipes and/or baby wipes, AP/Deo wipes, Sun care wipes, After-Sun treatment wipes, Insect repellent wipes, Body care and/or Body cleansing Wipes, Depilatory wipes, Make up removal wipes, Anti Acne wipes, sponges (e. g. polyurethane sponges) for all before mentioned applications , plaster for all before mentioned applications, serviettes/napkins, hand or kitchen towels as well as pet care wipes, e.g. for conditioning, cleansing or caring of the pet.

The emulsions according to the invention are suited for the application on home care wipes, e.g. for polishing, cleaning and the like. These wipes are typically used for household surfaces, such as e.g. furniture, surfaces in sanitary area, kitchen or technical areas.

The application of the emulsion onto the substrate is conducted according to techniques known it the art. Due to the rheological profile, the emulsions according to the invention can easily be applied to various substrates at temperatures below 40 °C

### EXAMPLES

Examples 1 and 2 are emulsions according to the invention. The amounts given are in weight-% based on the total weight of the emulsion

| **Component** | **Example 1** | **Example 2** |
|---|---|---|
| INCI name | | |
| Polyglycerol-2-dipolyhydroxystearate | 4,0 | 4,0 |
| Lauryl Glucoside | 4,0 | 4,0 |
| Sodium Stearoyl Glutamate | 0,1 | 0,1 |
| Mineral Oil | 35,0 | 35,0 |
| Isopropylpalmitate | 26,2 | 24,6 |
| Sun flower oil | - | 1,6 |
| Glycerol | 3,0 | 3,0 |
| Cetearyl alcohol | 3,0 | 3,0 |
| Chamomilla Recutica (Matricaria) Flower Extract | 0,1 | 0,1 |
| Aloe Barbadensis Leaf Extract | 0,1 | 0,1 |
| Citric acid | 0,09 | 0,09 |
| Water | 23,9 | 23,9 |
| Perfume | q.s. | q.s. |
| Preservatives | q.s. | q.s |
| | | |

The emulsions of examples 1 and 2 display very good sensory properties show stability at room temperature as well as at elevated temperature (30°C). In addition they display a high degree of whiteness, even though they contain amounts of coloured plant extracts.

## Claims

1. O/W emulsion containing
(a) at least one polyol poly-12-hydroxystearate
(b) at least one further non-ionic emulsifier
(c) at least one anionic co-emulsifier
(d) oil, where at least 75 weight-% of the oil has a polarity of ≥ 20 mN/m
(e) 10 to 30 weight -% water based on the total weight of the emulsion
(f) optionally at least one consistency regulator
(g) optionally at least one humectant
- whereas the total amount of emulsifiers [=(a) plus (b) plus (c)] is between 4 and 20 weight-%, preferably between 6 and 16 weight-%, based on the total weight of the emulsion.

2. Emulsion as claimed in claim 1, **characterized in that** anionic co-emulsifier (c) is present in an amount of 1 to 20 weight-% based on the amount of further non-ionic emulsifier (b).

3. Emulsion as claimed in any preceding claim **characterized in that** the weight ratio of polyol poly-12-hydroxystearate (a) to further non-ionic emulsifier (b) is in the range of 1:0,5 to 1:2.

4. Emulsion as claimed in any preceding claim, **characterized in that** the HLB value of polyol poly-12-hydroxystearate (a) is ≤ 10, preferably ≤ 8, more preferably ≤ 5.

5. Emulsion as claimed in any preceding claim, **characterized in that** the HLB value of the further non-ionic emulsifiers (b) is ≥ 10, preferably ≥ 12, preferably between 12 and 20.

6. Emulsion as claimed in any preceding claim, **characterized in that** the polyol-poly-12-hydroxystearate (a) is a polyglycerol-poly-12-hydroxy-stearate.

7. Emulsion as claimed in any preceding claim, **characterized in that** the further non-ionic emulsifier (b) is selected from the group consisting of
o (b-1) products of the addition of 2 to 50 mol ethylene oxide and/or 0 to 20 mol propylene oxide onto linear fatty alcohols containing 8 to 40 carbon atoms, onto fatty acids containing 12 to 40 carbon atoms and onto alkylphenols containing 8 to 15 carbon atoms in the alkyl group
o (b-2) C_{12/18} fatty acid monoesters and diesters of addition products of 1 to 50 mol ethylene oxide onto glycerol;
o (b-3) glycerol mono- and diesters and sorbitan mono- and diesters of saturated and unsaturated fatty acids containing 6 to 22 carbon atoms and ethylene oxide addition products thereof;
o (b-4) alkyl and/or alkenyl polyglycosides, preferably alkyl mono- and oligo glycosides containing 6 to 22 carbon atoms in the alkyl group and ethoxylated analogs thereof;
o (b-5) partial esters based on linear, branched, unsaturated or saturated C₆₋₂₂ fatty acids, ricinoleic acid and 12-hydroxystearic acid and glycerol, polyglycerol, pentaerythritol, dipentaerythritol, sugar alcohols, alkyl glucosides and polyglucosides; or
o (b-6) polysiloxane/polyalkyl/polyether copolymers and corresponding derivatives;
o and mixtures thereof

8. Emulsion as claimed in any preceding claim, **characterized in that** the further non-ionic emulsifier (b) is at least one alkyl and/or alkenyl polyglycoside.

9. Emulsion as claimed in any preceding claim, **characterized in that** the anionic co-emulsifier (c) is selected from the group of phosphate-, sulphate and carboxylate emulsifiers.

10. Emulsion as claimed in any preceding claim, **characterized in that** the anionic co-emulsifier (c) is a carboxylate emulsifier, preferably an acylglutamate.

11. Emulsion as claimed in any preceding claim, **characterized in that** the anionic co-emulsifier (c) is present in an amount of 0,01 to 10, preferably 0,05 to 5 weight-%, preferably 0,03 to 1,4 weight-% based on the total weight of the emulsion.

12. Emulsion as claimed in any preceding claim, **characterized in that** the oil (d) is present in an amount of 40 to 70 weight-% based on the total weight of the emulsion.

13. Emulsion as claimed in any preceding claim, **characterized in that** at least 80, preferably at least 90 weight-% of the oil (d) has a polarity of ≥ 20 mN/m.

14. Emulsion as claimed in any preceding claim, **characterized in that** at least 30 weight-%, preferably at least 40 weight-%, more preferably at least 50 weight- %, more preferably at least 60 weight-% of the oil (d) has a polarity of ≥ 35 mN/m.

15. Emulsion as claimed in any preceding claim, **characterized in that** the oil (d) comprises at least one hydrocarbon-based oil, preferably mineral oil.

16. Emulsion as claimed in claim 15, **characterized in that** the hydrocarbon-based oil (d) constitutes at least 40 weight-%, more preferably at least 50 weight-% of the oil (d), based on the total weight of the oil.

17. Emulsion as claimed in any preceding claim, **characterized in that** the oil (d) comprises an oil which is selected from the group consisting of liquid synthetic triglyceride mixtures; vegetable oils; guerbet alcohols; liquid linear or branched esters; liquid substituted cyclohexanes; symmetric or asymmetric dialk(en)ylethers having from 6 to 22 C atoms per alk(en)yl group; linear or branched dialk(en)ylcarbonates derived from C 6 to 22 fatty alcohols; ring-opening products of epoxidized fatty acid esters and polyols; silicone oils and mixtures thereof.

18. Emulsion as claimed in any preceding claim, **characterized in that** the oil (d) has a viscosity of 1 to 100 mPa•s, preferably ≤ 50 mPa•s; measured with a Höppler falling sphere viscosimeter at 20°C (method DGF C-IV 7).

19. Emulsion as claimed in any preceding claim, **characterized in that** at least one cosmetic agent is additionally present.

20. Emulsion as claimed in any preceding claim, **characterized in that** the emulsion has a viscosity of 100 to 10,000 mPa•s at 25°C, preferably of 500 to 4,000 measured according to Brookfield RVF, spindle 3, 10 rpm.

21. O/W Emulsion, **characterized in that** it contains
ix) 3 to 7 weight-% of polyol-12-poly-hydroxystearate (a), preferably polyglycerol-12-poly-hydroxystearate
x) 3 to 7 weight-% of a further non-ionic emulsifier (b), preferably at least one alkyl and/or alkenyl polyglycoside
xi) 0.1 to 10 weight-% of at least one anionic co-emulsifier (c)
xii) 40 to 70 weight-% of an oil component (d), whereas at least 75 weight-% of the oil, has a polarity of ≥ 20 mN/m;
xiii) 10 to 30 weight-%, preferably 20 to 25 weight % of water (e)
xiv) 0-5 weight-% of at least one consistency regulator (f)
xv) 0 -5 weight % of at least one humectant (g)
xvi) 0-5 weight % of at least one cosmetic agent (h)
- whereas the total amount of emulsifiers [=(a) plus (b) plus (c)] is between 6,1 and 16 weight-%, based on the total weight of the emulsion

22. Use of an emulsion as claimed in any of the preceding claims for the preparation of a cosmetic composition.
